Europäisches Patentamt

European Patent Office

Office européen des brevets

(11) Publication number: **0 492 007 B1**

## EUROPEAN PATENT SPECIFICATION

(45) Date of publication of patent specification: **08.02.95**　(51) Int. Cl.⁶: **B01J 13/04**, A01N 25/28

(21) Application number: **90313014.4**

(22) Date of filing: **29.11.90**

(54) **Process for producing liquid loaded powders.**

(43) Date of publication of application:
**01.07.92 Bulletin 92/27**

(45) Publication of the grant of the patent:
**08.02.95 Bulletin 95/06**

(84) Designated Contracting States:
**AT BE CH DE DK ES FR GB GR IT LI LU NL SE**

(56) References cited:
**EP-A- 0 322 137**
**FR-A- 2 185 390**
**GB-A- 2 151 202**

(73) Proprietor: **F.H. FAULDING & CO. LIMITED**
**160 Greenhill Road**
**Parkside**
**South Australia 5063 (AU)**

(72) Inventor: **Cline, John F.**
**223 Massachusetts Street**
**Westfield**
**NJ 07090 (US)**
Inventor: **Nichols, Larry D.**
**134 Gray Street,**
**Arlington MA 02174 (US)**

(74) Representative: **Jenkins, Peter David et al**
**PAGE WHITE & FARRER**
**54 Doughty Street**
**London WC1N 2LS (GB)**

Rank Xerox (UK) Business Services
(3.10/3.09/3.3.3)

EP 0 492 007 B1

**Description**

The present invention concerns liquid loaded powders, a process for their production and compositions containing the powders.

A liquid loaded powder in general comprises microporous particles, the micropores of which contain a liquid load.

FR-A-2,185,390 discloses powders comprising microencapsulated medicament. Each microcapsule comprises a particle having an outer generally porous shell of coating agent with an inner core of fine particles.

United States Patent No. 4029726 discloses the formation of polymer liquid composites that may be produced in the form of films, fibres or microspheres. The polymer liquid composites are formed by dissolving a strong rigid high molecular weight polymer in a solvent that is functionally and sterically capable of strong bonding to the polymer chain followed by reduction of the solvent power at temperatures low enough to avoid thermal sintering or coalescence of the polymer.

The specification states that the basic criteria are met by cellulosic polymers, strong polar solvents and precipitation of the cellulosic polymer from a solution in the solvent by rapid displacement of the solvent by a miscible nonsolvent. The cellulosic polymers described include cellulose triacetate in which the degree or substitution is greater than 2.9, cellulose nitrate in which the degree of substitution is greater than 2.9 and cellulose tripropionate.

Suitable solvent non-solvent systems include acetic acid/water, acetic acid/isopropanol, methylene chloride/methanol, methylene chloride/isopropanol.

Microspherical products in the size range from 1 to 500 microns can be prepared by emulisifying the polymer solution in a miscible non-solvent followed by coagulation of the suspension using a liquid which does not dissolve the polymer, is miscible with the polymer solvent and is also miscible with the liquid used as the suspension medium.

Alternatively microspheres can be prepared by spraying the polymer solution into a miscible non-solvent. Useful liquid loads can then be impregnated into the microspheres by liquid phase interchange.

The method of preparing a liquid loaded powder described in United States Patent No. 4029726 can result in contamination of the liquid load or alternatively greater production costs in order to avoid the risk of contamination. Furthermore since the liquid load is not directly introduced into the powder the process is potentially less economic than a direct method of loading the powder.

An aim of the present invention is to overcome some or all of these disadvantages.

Accordingly the present invention provides a process for producing a liquid-loaded powder that is capable of releasing its liquid load when subjected to shearing forces, which process comprises dissolving a cellulosic polymer and a liquid load in a polar solvent to form a solution and dispersively evaporating the solvent from the solution to form a liquid-loaded powder characterized in that the solvent is dispersively evaporated from the solution by spraying the solution into a tower and passing heated air or an inert gas through the tower from an entry to an exit at a rate and at an entry temperature sufficient to maintain the air or inert gas leaving the tower at an exit temperature above the dew point of the solvent.

The polar solvent may be a volatile solvent such as a low molecular weight ester or diester. Preferred volatile polar sovents include methylene chloride, chloroform, ethanol, acetone, ethyl acetate, ethyl carbonate, methyl formate and mixtures thereof.

The cellulosic polymer may comprise a cellulose acetate, a cellulose butyrate, a cellulose nitrate, a cellulose propionate, an ethyl cellulose, or discrete or molecular mixtures thereof.

The selection of solvent is dependent upon the polymer selected and is also partially dependent upon the proposed liquid load. Preferably the solvent has a boiling point that is significantly lower than that of the proposed liquid load. However in the event that the proposed liquid load has a boiling point close to that of the proposed solvent, simultaneous evaporation of the liquid load can be avoided by causing the evaporation to take place in an atmosphere saturated with the vapour phase of the proposed liquid load.

Methylene chloride is the preferred solvent for cellulose triacetate and ethanol is the preferred solvent for an ethyl cellulose. However, methylene chloride or a mixture of methylene chloride and ethanol may also be used as solvents for ethyl cellulose.

In order to obtain best results the conditions maintained inside the tower are such that the solvent progressively evaporates as droplets of the solution proceed from the point of entry to the point of collection. The rate of evaporation of the solvent can be controlled by controlling the temperature inside the tower and the proportion of the solvent present in the atmosphere. When using methylene chloride as solvent the atmosphere inside the tower may contain from 0 to 40% by volume of methylene chloride. The solution may be sprayed from the top or bottom of the tower, co-current or counter current to a stream of heated air or inert gas passed through the tower. The tower may have one or more spraying nozzles. The air or inert gas may be passed through the tower at an average velocity in the range from 0.3 to 20 cubic metres per minute for a tower having a diameter of 1 metre. If the solvent or liquid load or both are flammable then an inert gas is preferred.

The particle size of the powder is controlled mainly by the size of the nozzle but can be controlled to some extent by the hydrostatic pressure and the viscosity of the solution and hence its temperature. The temperature near the spray in a spray-drying tower also plays a part in determining particle size. When using a volatile solvent such as methylene chloride, the temperature near the spray is preferably maintained in the range from 30°C to 95°C.

Ideally the relative humidity of the gas exiting the tower is maintained at less than 40% in order to avoid the presence of excessive moisture in the powder.

Powders produced by this process may comprise up to 95% by weight of the liquid load. The proportion of liquid loaded into the powder depends upon the desired end use and may for example be as low as 20%. However normally the liquid load comprises from 50 to 95% by weight of the loaded powder.

The temperature at the coolest point in the tower is maintained at a level above the dew point of the solvent otherwise it tends to liquefy and be reabsorbed by the powder.

As a result of the control that can be achieved over the particle size of the powder, it is possible to produce powders comprising particles having an average particle size in the range from 5 to 100 microns and in which the proportion of particles having a particle size greater than 150 microns is negligible. The particles comprising the powders of the invention are usually microporous and have interconnecting pores ranging in size from 10 to 5000 Angstroms.

The powders of the present invention may be used to produce shaped articles such as those described in co-pending application of publication number EP-A-400 910. In particular cakes of soap containing powders loaded with perfume and other useful additives can be produced using the methods disclosed in co-pending patent application of publication number EP-A-400 910. Cakes of soap containing liquid loaded powders of the present invention may be made by conventional means as well. The liquid loaded powder is mixed with the raw soap at the stage when fragrances and other additives are normally incorporated. However care needs to be taken to avoid the application of shearing forces to the liquid loaded powder. Unlike normal soap formulations soap containing liquid loaded powders of the present invention release their load when the powder is subjected to the normal action of washing.

In addition even when soap containing a liquid loaded powder is not in use, the liquid load contained in the powder migrates from the powder into the matrix of soap until the soap is saturated and equilibrium established. As the liquid load evaporates from the soap, additional liquid load migrates from the powder to maintain the equilibrium. In this way the fragrance of a soap can be maintained for a much longer period than with conventional formulations.

Other liquid loads for the powders of the invention include;

sunscreen agents;

skin moisturizers;

flavourings for such products as toothpaste;

antiperspirants;

anti-fungal agents for foot powders;

perfumes;

baby oil;

a mixture containing a sunscreen, a moisturizer and a fragrance for use in cosmetic foundations;

topical analgesics;

other cosmetic products;

other topical OTC products; and

medicines for oral administration.

In general the liquid loaded powders of the present invention may be used wherever a liquid, cream, stick, ointment, cake or powder is currently used to deliver substances to the skin. The liquid load may be liquid, mixtures of liquids, solids dissolved in liquids etc. A liquid loaded powder of the invention may be readily mixed with other types of liquid loaded powder, other powders, pigments, pure fragrances, oils and other additives.

A liquid loaded powder of the present invention may also be gently blended with a liquid to form a "particle-stabilized emulsion" which can provide long term stability without the use of chemical emulsifiers, surfactants or stabilizers. The particles of liquid loaded powder retain the ingredients in a dispersed state and prevent them from separating into layers and yet the blend can approach the softness of a liquid/liquid emulsion. Creams, lotions and ointments may be prepared in this way. In this case the liquid may be a carrier or diluent and an active ingredient may be contained as the liquid load in the liquid loaded powder or the active ingredient may be contained in the dispersing liquid or in both liquids.

Preferred embodiments of the invention are described with reference to the accompanying Examples.

Example 1

15 gm of CTA was dissolved in 900 gm of methylene chloride aided by moderate stirring for 1 hour. 85 grm of Fragrance "A" was added to the resulting clear solution and stirred until fully dispersed. Fragrance "A" is a highly concentrated test fragrance composed of 20.5% Aldo-66, 6.9% DC-200 Dimethicone, 20.6% DC-345 Cyclomethicone and 52% Firmenich Fragrance Oil 423-236/B. The solution was sprayed at 7MPa from a 0.343 cm nozzle downward into a tower 100 cm in diameter and 300 cm tall through which 1250 litres per minute of solvent-free air was passing from top to bottom.

The resulting evaporatively formed, liquid-containing CTA powder was collected on a fabric filter spanning the bottom of the tower, and the solvent-laden air was passed through carbon beds to collect solvent vapours.

The product was transferred from the filter into a steel tray and left exposed as a 1 cm layer in a ventilated hood for 15 minutes to remove residual solvent. Analysis showed 85.2 percent fragrance and 14.8 percent CTA, with less than 1 ppm of methylene chloride.

A 5 mg sample of this powder placed on the inner left wrist and rubbed gently with the right forefinger was readily distributed over an area of about 3 square centimetres and then completely vanished on further gentle rubbing. Fragrance imparted to the treated skin area persisted until washed after six hours.

No grittiness was observed during this experiment; the mean particle diameter was about 30 microns, and particles larger than 150 microns were not observed.

Example 2 Baby Oil Preparation

39.15 grm of CTA was dissolved in 520.4 grm of methylene chloride by stirring for 1 hour. 223.3 grm of mineral oil was then mixed with a further 3234.6 grm of methylene chloride and the two solutions mixed. The resulting lacquer was sprayed at 6.6 MPa from a 0.0343 cm nozzle downwardly into a tower 100 cm in diameter, and 300 cm tall through which 2300 litres per minute of air was passing from top to bottom. The temperature at the top of the tower measured 48.1 °C and at the base 24.0 °C.

The powder was collected in a bin and subsequently placed in a steel tray for drying and left exposed as a 1 cm layer in a ventilated hood for 15 mins to remove residual solvent. The solvent outlet stream was passed through a series of carbon beds.

Product Description

The mean particle size diameter of the resulting particles was 27.5 microns and 90% of the particles had a diameter below 57.5 microns. The powder is white and fluffy and has very good rub out characteristics i.e. the powder disappeared into an imperceptible film when rubbed gently on the skin. When a quantity of the powder was squeezed together in the hand, a semi-solid mass resulted, but could be easily broken down again into its powder form.

Example 3A Topical Analgesic

The procedure of Example 2 was used to prepare a topical analgesic. 40 grm of CTA was dissolved in 531.4 grm of methylene chloride. A mixture of 110 grm of phenyltrimethicone (DC 556), 20 grm menthol and 30 grm methyl salicylate was then dissolved in 1768.6 grm of methylene chloride and the two solutions combined. The resultant solution was spray dried as in Example 1. The conditions used during the spray drying process are set out below.

| Air flow | 3100 litres per minute |
|---|---|
| Pump pressure | 7 MPa |
| Flow Rate | 13 litres of lacquer per hour |
| Top Temp | 50.75 °C |
| Bottom Temp | 43.15 °C |
| Rel. Humidity of tower | 35% |

Product Description

The resulting product was white and had a smooth feel and a strong menthol odour. The phenyltrimethicone imparts a "silky, smooth" feel to the resultant powder. The mean particle size was 30.2 $\mu$m with 90% of the particles being less than 55.5 $\mu$m. The active ingredient constituted approximately 25% of the end product.

Example 3B Topical Analgesic

The procedure of Example 3A was followed substituting the following materials:
40 grm CTA
531.4 grm methylene chloride
10 grm phenyltrimethicone
60 grm menthol
90 grm methylsalicylate
1768.6 grm methylene chloride

Processing conditions remain identical to that of Example 3A, but in this case the active ingredient constituted 75% of the end product. The powder looked and felt identical to the powder of Example 3A. It did however have a more potent menthol odour and the "heating sensation" associated with the methyl salicylate ingredient was felt in a far shorter period of time in comparison with the product of Example 3A.

Example 4 Toothpaste Flavourings

The procedure of Example 2 was used to produce a flavouring compound suitable for use in a toothpaste formulation. 35.3 grm CTA was dissolved in 469 grm of methylene chloride and 319.7 grm of peppermint flavouring was dissolved in a further 2967 grm of methylene chloride. The two solutions were then combined and spray dried using the conditions set out below.

| Conditions: | |
|---|---|
| Air flow | 2550 litres/minute |
| Pump pressure | 6.4 MPa |
| Flow Rate | 13 litres per hour |
| Top temp | 34.6 °C |
| Bottom temp | 19.0 °C |

The white fluffy powder had a strong peppermint odour and flavour.

Example 5A Sunscreen Preparation

35 grm of CTA was dissolved in 465.2 grm methylene chloride. The remaining ingredients:
- 32.6 grm Parsol MCX®
- 21.8 grm Sunarome WMO®
- 340.0 grm phenyltrimethicone

were dissolved in 3016 grm methylene chloride and the two solutions mixed. The combined solutions were spray dried using the conditions set out below:

| Conditions: | |
|---|---|
| Air flow | 1560 litres per minute |
| Pump pressure | 8 MPa |
| Top temp | 30.5 °C |
| Bottom temp | 17.3 °C |
| Mean particle size | 38.1 microns with 90% less than 78.9 microns |
| Nozzle size | 0.0343 cm diameter |
| Nozzle type | Small, single fluid pressure/pneumatic |

Parsol MCX® is a trade name for octyl methoxycinnamate and is a known UV-B blocker.

Sunarome WMO® is a trade name for an octyl salicylcate product and is a known UV blocker.

Phenyltrimethicone is an occulsive-type moisturizing agent which prevents moisture from leaving the skin surface.

Example 5B Sunscreen Powder

200 grm of commercial suntan oil (sun protection Factor 6) was blended with 2877 grm of methylene chloride and 35 grm of CTA dissolved in a further 465 grm of methylene chloride. The resulting lacquers were mixed and spray dried under the conditions described in Example 5A. The resulting powder was dermatologically tested for efficacy against 100 grm of the commercial blend alone and was found to be equally effective when 117.8 mg of product (100 mg active) was applied to the skin. The powder form blended into the skin very easily, had none of the disadvantages associated with suntan oil and remained on the skin for a considerable period of time.

Example 5C

201.1 grm of commercial suntan oil (SPF 4) and 2877 grm of methylene chloride were mixed. 35 grm of CTA was dissolved in a further 465 grm of methylene chloride. Both solutions were mixed and the resultant lacquer spray dried under the conditions described in Example 5A.

Efficacy tests on 100 mg of the commercial oil and 117.6 mg of powder (100 mg active) gave readings of true SPF values at 3.7 and 4.1.

Example 6 Soap Bar

A commercial blend of natural soap stick containing sodium tallowate and sodium cocoate in an 80:20 ratio was milled and ground several times to produce a dough. The mixture was put through an auger and extruded several times during which time a 4% pure perfume oil was added along with 5% cellulosic powder previously produced containing 80% perfume oil, as the active ingredient. The extrudate was put into a long bar mould and when solidified was chopped into small bars. Alternatively, the extrudate can be compacted into individual bars or moulds.

Product Description

Comparisons were made between a soap bar with pure perfume oil (4%) and a soap containing perfumed liquid loaded powder in addition to pure perfume oil (8%). No visual differences in either product were noted. Sudsing and cleansing properties in both were equal. Identical measurements were found on testing with a penetrometer although the soap containing the liquid-loaded powder contained a total of 8% perfume and soap is usually saturated at 4% with penetrometer readings decreasing with each additional percent increase in oil. There was a marked difference in the strength of fragance noted from the two bars.

Example 7 Perfumed Powder with Pigment

A perfumed pigmented powder was prepared using the method described in Example 2. The ingredients and the spray drying conditions are set out below:

Ingredients

136.3 grm CTA
1811.2 grm methylene chloride
1239.15 grm methylene chloride
783.8 grm Perfume R
0.13 grm Brown LS-553 Tricon Colour Dye

| Spray Drying Conditions | |
|---|---|
| Top temp | 26.2 °C |
| Bottom temp | 7.5 °C |
| Air flow | 1600 litres per minute |
| Pump pressure | 8.2 MPa |
| Residual $H_2O$ | 0.12% |
| Nozzle | 0.0343 cm |
| Flow Rate | 13 litres per hour |

Product Description

A light fluffy brown powder resulted with a perfume which persisted for several hours after application to the skin. The powder contained 85% by weight of perfume.

Example 8 Anti-Fungal Foot Powder

## Ingredients:

57 grm CTA                    )
757 grm methylene chloride )        Mix 1
3 grm Tolnafate              )
1143 grm methylene chloride)        Mix 2

| Conditions | |
|---|---|
| Air flow | 2550 litres/minute |
| Pump pressure | 7 MPa |
| Flow Rate | 13 litres/hr |
| Top temperature | 45 °C |
| Bottom temperature | 25 °C |
| Nozzle diameter | 0.0343 cm |

The ingredients of Mix 1 and Mix 2 are blended together and the resulting lacquer spray dried under the above conditions. The white fluffy product contained 5% tolnafate as the active ingredient. Foot powder in this form is believed to retard wash-off by moisture from the foot.

Example 9 Moisturising agent

A moisturising formulation for topical application was prepared using the method of Example 2. the ingredients and the spray drying conditions used are set out below:

Ingredients

252 grm CTA
3348 grm methylene chloride
1428 grm Propylene Glycol
21852 gr methylene chloride

| Spray Drying Conditions | |
| --- | --- |
| Top temp | 37.3°C |
| Bottom temp | 14.0°C |
| Air flow | 1840 litres per minute |
| Pump Pressure | 2.1 MPa |
| Mean particle size | 45.5 μm with 90%<94.0 μm |
| Residual water | 10.6% |
| Nozzle size | 0.137 cm |
| Flow rate | 53 litres per hour |

Description

The resulting product contained 85% by weight of moisturiser and had noticeably larger particles than the previous examples and the product was "sticky" to feel. This was probably as a result of the high residual water content in the end product due to the propensity of propylene glycol to absorb water. It is a known humectant type moisturizer.

The particles produced by this method were of an egg-shell type. i.e. a shell of CTA surrounding the moisturiser.

Example 10

One part by weight of ethyl cellulose and 9 parts by weight of DC-345 were dissolved in 90 parts by weight of ethylacetate. In order to avoid precipitation and possible incomplete redissolution of the polymer and the silicone, the ethyl cellulose and the DC-345 were dissolved separately in approximately equal portions of the solvent and the two solutions were then blended and thoroughly mixed. The solution was pumped at a pressure of 7 MPa and sprayed through a 0.066 cm carbide nozzle mounted downwardly in the top of a spray drying tower. Nitrogen at an inlet temperature of 100°C was passed through the tower at a flow rate in the range from 1000 to 2000 litres per minute. The flow rate was adjusted in order to maintain an exit temperature of 10 to 20°C. The tower dimensions were similar to those described in Example 1.

Other than fragrance the product analysed between 81 and 86% DC-345 and could not be distinguished by appearance, feel or behaviour from the product of Example 1.

Example 11

The experiment of Example 8 was repeated using acetone as the solvent. The product analysed between 83 and 87% DC-345 but showed some aggregation. Flakes of powder separated from the walls of the spray dry tower tended to remain coherent unless subjected to gentle compression. In other respects its appearance and behaviour on the skin were like those of the powder produced in Example 1.

Example 12

A liquid-containing, cellulosic powder formulation was prepared using the methodology of Example 1 but substituting ethyl cellulose (Dow Chemical Ethocel 100 Premium) for the CTA and nonvolatile Dow Corning 556 Silicone Fluid for Fragrance "A". The spray solution, 30 grams of polymer and 70 grams of silicone liquid in 900 grams of methylene chloride, was sprayed downwardly from the top of a tower and evaporatively formed, liquid-containing powder was collected at the bottom. After loss of volatiles, analysis confirmed that the powder contained 70% silicone fluid.

Example 13

Another example of liquid containing powder was prepared according to the methodology of Example 12, except for the use of cellulose propionate (Aldrich Chemical Corporation No. 33,018-3, M.W. 75,000, Flow Temperature 356°F) in place of ethyl cellulose. The product analyzed to contain 70% DC-556 silicone fluid.

Example 14 - Insect Repellent Composition

A liquid-loaded powder was prepared by spray evaporative drying. A liquid payload was prepared from 60 parts by weight of N, N-diethyl-m-toluamide (DEET) and 40 parts by weight of 2-octyl dodecanol, a heavy secondary alcohol commonly used in cosmetic formulations. 40 grams of cellulose triacetate was dissolved in 3000 grm of methylene chloride by moderate stirring for 4 hours. To that solution was added 460 grm of the previously prepared payload diluted with 1000 grm of methylene chloride. The resulting homogeneous solution was sprayed at 7 MPa from a 0.0343 cm nozzle, downwardly into a tower 100 cm in diameter and 300 cm tall, through which 1250 litres per minute of solvent-free air was passing from top to bottom.

The evaporatively-formed powder was collected on a fabric filter spanning the bottom of the tower, and the solvent-laden air was passed through carbon beds to collect and recover solvent. The product was transferred to a steel tray and exposed as a 1 cm deep layer in a ventilated hood for 25 minutes to remove residual solvent. Analysis showed 8% cellulose triacetate, 36.8% octyldodecanol, and 55.2% DEET, with less than 5 ppm of residual methylene chloride.

The resulting white powder could be readily dusted onto the skin and made to liquefy and vanish by gentle rubbing. There was no perceptible grit or gumminess. When an adult volunteer self-administered 50 mg of powder, equivalent to about 27 mg of DEET, to his forearm, exposure of the forearm to about 100 mosquitoes in a 0.1 cubic metre chamber for 5 minutes resulted in only two observable bites. A similar experiment with an untreated forearm produced over 50 bites.

Example 15

The powder of example 14 was applied at a level of about 1 mg per 10 cm to a circular disk of paper 10 cm in diameter, which was subsequently placed on the floor of a 5 litre glass jar containing about 20 disease-free deer ticks. At the end of five minutes the paper, which covered about one half of the floor area, was tick free.

Example 16

A powder like that of example 14 was prepared using as a payload pure 2-ethyl-1,3-hexanediol (6-12) insect repellent. The powder analyzed to contain 8.1% cellulose acetate and 91.9% 6-12 liquid, with less than 1ppm of methylene chloride. Application to a forearm precisely as in example 14 led to 3 bites under conditions in which an untreated control forearm suffered about 15.

Apparatus suitable for use in forming the liquid loaded powders of the present invention will now be described with reference to the accompanying drawing.

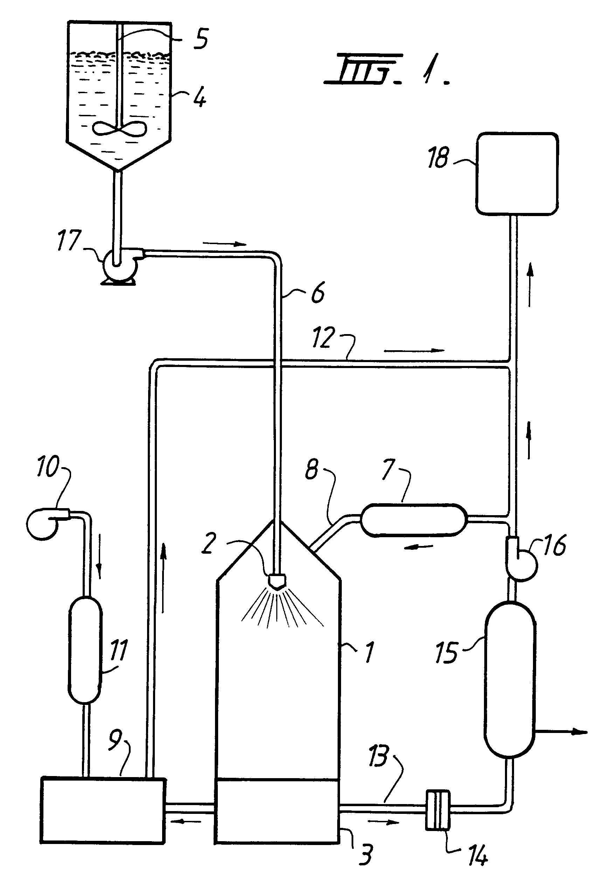

Figure 1 is a flow diagram and depicts a spray tower 1 having a nozzle 2 located near its top and a powder collector tray 3 at the bottom. The apparatus for producing the liquid loaded powders of the invention also includes a conventional mixing vessel 4 having an electrically driven stirrer 5. The mixing vessel is connected to the nozzle 2 by means of pipeline 6.

The liquid load and cellulose triacetate are dissolved in methylene chloride in the mixing vessel 4 by use of stirrer 5. The solution is pumped through pipeline 6 by means of pump 17 to the nozzle 2. Air heated by heater 7 is blown into the top of the tower through duct 8. The heated air causes the methylene chloride to evaporate from the droplets formed by the nozzle 2 resulting in the formation of liquid loaded powder which is collected at the bottom of the tower in the collector tray 3. Powder collected from the tray 3 is taken to a vessel 9. Dry air is blown by means of blower 10 through heater 11 to vessel 9. Residual solvent extracted from the powder is blown with the air into a carbon bed through duct 12.

Air blown into the tower through duct 8 leaves the tower through duct 13 and is passed through a filter 14 and a condenser 15. The condenser 15 is cooled in order to condense methylene chloride from the air for recycling back to the mixing vessel 4. Most of the air from which methylene chloride has been condensed is then blown by means of blower 16 back through heater 7 and duct 8 into the spray tower 1. Excess air is bled from the system through a carbon bed 18 and exhausted to atmosphere. The carbon bed removes hydrocarbons still entrained in the air.

**Claims**

1.  A process for producing a liquid-loaded powder that is capable of releasing its liquid load when subjected to shearing forces, which process comprises dissolving a cellulosic polymer and a liquid load in a polar solvent to form a solution and dispersively evaporating the solvent from the solution to form a liquid-loaded powder characterized in that the solvent is dispersively evaporated from the solution by spraying the solution into a tower and passing heated air or an inert gas through the tower from an entry to an exit at a rate and at an entry temperature sufficient to maintain the air or inert gas leaving the tower at an exit temperature above the dew point of the solvent.

2.  A process according to claim 1 wherein the solvent is volatile and the process is further characterized by maintaining the entry temperature in the range from 30°C to 95°C.

3.  A process according to claim 1 or claim 2 further characterized by maintaining a relative humidity of less than 40% in gases exiting from the tower.

4.  A process according to claim 2 wherein the polar solvent is a low molecular weight ester or diester.

5.  A process according to claim 2 wherein the polar solvent is selected from the group consisting of methylene chloride, chloroform, ethanol, acetone, ethyl acetate, ethyl carbonate, methyl formate and mixtures thereof.

6.  A process according to claim 1 wherein the cellulosic polymer is selected from the group consisting of cellulose acetates, cellulose butyrates, cellulose nitrates, cellulose propionates, ethyl celluloses and discrete or molecular mixtures thereof.

7.  A process according to claim 2 wherein the cellulosic polymer is cellulose triacetate and the polar solvent is methylene chloride.

8.  A process according to claim 2 wherein the cellulosic polymer is ethyl cellulose and the polar solvent is selected from the group consisting of ethanol, methylene chloride and mixtures thereof.

9.  A process according to claim 7 wherein the tower has an atmosphere containing from 0 to 40% by volume of methylene chloride.

10. A process according to claim 1 wherein the liquid load comprises up to 95% by weight of the liquid loaded powder.

11. A process according to claim 10 wherein the liquid load comprises from 50 to 95% by weight of the liquid loaded powder.

12. A process according to claim 8 wherein the solution is sprayed through one or more nozzles downwardly from the top of the tower.

13. A process according to claim 1, wherein the powder comprises microporous particles having a particle size in the range from 1 to 500 microns and pores ranging in size from 1 to 500 nanometers, the pores being capable of holding the liquid load.

14. A process according to claim 13, wherein the particles have an average particle size in the range from 5 to 100 microns and the proportion of particles having a size greater than 150 microns is negligible.

15. A process according to claim 13 or claim 14, further comprising the step of forming the liquid-loaded powder into a cake of soap.

16. A process according to claim 13 or 14, wherein the liquid load contains a pharmaceutical, a sunscreen agent, a skin moisturizer, a tooth paste flavouring compound, an antiperspirant, an antifungal agent for use on feet, a fragrance, a baby oil or a topical analgesic.

17. A process according to claim 15, wherein the liquid load contains a preparation for oral administration.

18. A process according to claim 13 or claim 14, further comprising the step of dispersing the liquid-loaded powder in a dispersing liquid.

19. A process according to claim 13 or claim 14, wherein the liquid load contains an insect repellent.

20. A process according to claim 19, wherein the insect repellent is chosen from N,N-diethyl-m-toluamide and 2-ethyl-1,3-hexanediol.

21. A process according to claim 19 or claim 20, wherein the composition further comprises an emollient not incorporated within the liquid load.

22. A processing according to claim 14, wherein the liquid load comprises a sunscreen preparation.

23. A process according to claim 22, wherein the liquid sunscreen preparation comprises a compound chosen from oxybenzone, amyl-p-diethylaminobenzoate, homomethyl salicylate, octyl salicylate, mono-p-aminobenzoate, octyl-methoxycinnamate, and 2-ethoxy ethyl-p-methoxycinnamate.

24. A process as defined in claim 22 or claim 23, further comprising the step of mixing an emollient with the liquid loaded powder.

25. A process according to claim 13 or claim 14, wherein an active ingredient is contained within the liquid load.

26. A process according to claim 18, wherein an active ingredient is contained within the dispersing liquid.

**Patentansprüche**

1. Verfahren zum Herstellen eines flüssigkeits-beladenen Pulvers, das seine Flüssigkeitsbeladung freigeben kann, wenn es Scherkräften unterworfen wird, welches Verfahren umfaßt: Auflösen eines Cellulosepolymers und einer Flüssigkeitsbeladung in einem polaren Lösungsmittel zur Bildung einer Lösung und dispergierendes Verdampfen des Lösungsmittels aus der Lösung zur Bildung eines flüssigkeits-beladenen Pulvers, dadurch gekennzeichnet, daß das Lösungsmittel aus der Lösung dispergierend verdampft wird, indem die Lösung in einen Turm gesprüht und Heißluft oder ein Edelgas durch den Turm von einem Einlaß zu einem Auslaß mit einer Geschwindigkeit und bei einer ausreichenden Einlaßtemperatur geleitet wird, um die den Turm verlassende Luft oder das Edelgas auf einer Auslaßtemperatur oberhalb des Taupunktes des Lösungsmittels zu halten.

2. Verfahren nach Anspruch 1, bei welchem das Lösungsmittel flüchtig ist und das Verfahren ferner dadurch gekennzeichnet ist, daß die Einlaßtemperatur im Bereich von 30°C bis 95°C gehalten wird.

3. Verfahren nach Anspruch 1 oder 2, dadurch gekennzeichnet, daß eine relative Feuchtigkeit von weniger als 40 % in den den Turm verlassenden Gasen aufrechterhalten wird.

4. Verfahren nach Anspruch 2, bei welchem das polare Lösungsmittel ein Ester oder Diester mit niedrigem Molekulargewicht ist.

5. Verfahren nach Anspruch 2, bei welchem das polare Lösungsmittel aus der Gruppe ausgewählt ist, welche besteht aus Methylenchlorid, Chloroform, Ethanol, Aceton, Ethylacetat, Ethylcarbonat, Methylformat und Mischungen derselben.

6. Verfahren nach Anspruch 1, bei welchem das Cellulosepolymer ausgewählt ist aus der Gruppe, welche besteht aus Celluloseacetaten, Cellulosebutyraten, Cellulosenitraten, Cellulosepropionaten, Ethylcellulosen und diskreten oder molekularen Mischungen derselben.

**7.** Verfahren nach Anspruch 2, bei welchem das Cellulosepolymer Cellulosetriacetat und das polare Lösungsmittel Methylenchlorid ist.

**8.** Verfahren nach Anspruch 2, bei welchem das Cellulosepolymer Ethylcellulose ist, und das polare Lösungsmittel aus der Gruppe ausgewählt ist, welche besteht aus Ethanol, Methylenchlorid und Mischungen derselben.

**9.** Verfahren nach Anspruch 7, bei welchem der Turm eine Atmosphäre aufweist, welche von 0 bis 40 Vol.-% Methylenchlorid enthält.

**10.** Verfahren nach Anspruch 1, bei welchem die Flüssigkeitsbeladung bis zu 95 Gew.-% des flüssigkeits-beladenen Pulvers beträgt.

**11.** Verfahren nach Anspruch 10, bei welchem die Flüssigkeitsbeladung von 50 bis 95 Gew.-% des flüssigkeits-beladenen Pulvers beträgt.

**12.** Verfahren nach Anspruch 8, bei welchem die Lösung durch eine oder mehrere Düsen vom oberen Ende des Turms nach unten gesprüht wird.

**13.** Verfahren nach Anspruch 1, bei welchem das Pulver mikroporöse Teilchen mit einer Teilchengröße im Bereich von 1 bis 500 $\mu$m und Poren im Größenbereich von 1 bis 500 nm aufweist, wobei die Poren zum Halten der Flüssigkeitsbeladung geeignet sind.

**14.** Verfahren nach Anspruch 13, bei welchem die Teilchen eine durchschnittliche Teilchengröße im Bereich von 5 bis 100 $\mu$m aufweist, und der Anteil von Teilchen mit einer Größe von mehr als 150 $\mu$m vernachlässigbar ist.

**15.** Verfahren nach Anspruch 13 oder 14, welches den weiteren Schritt umfaßt, daß das flüssigkeits-beladene Pulver zu einem Seifenkuchen gebildet wird.

**16.** Verfahren nach Anspruch 13 oder 14, bei welchem die Flüssigkeitsbeladung ein pharmazeutisches Sonnenschutzmittel, einen Hautbefeuchter, eine Zahnpasta-Geschmacksverbindung, ein Antitranspirationsmittel, ein pilzverhinderndes Mittel für die Verwendung an den Füßen, ein Duftmittel, ein Babyöl oder ein lokales Analgetikum enthält.

**17.** Verfahren nach Anspruch 15, bei welchem die Flüssigkeitsbeladung ein Präparat zur oralen Verabreichung enthält.

**18.** Verfahren nach Anspruch 13 oder 14, welches den weiteren Schritt umfaßt, daß das flüssigkeits-beladene Pulver in einer Dispersionsflüssigkeit dispergiert wird.

**19.** Verfahren nach Anspruch 13 oder 14, bei welchem die Flüssigkeitsbeladung ein Insektenabwehrmittel enthält.

**20.** Verfahren nach Anspruch 19, bei welchem das Insektenabwehrmittel ausgewählt ist aus N,N-Diethyl-m-Toluamid und 2-Ethyl-1,3-Hexandiol.

**21.** Verfahren nach Anspruch 19 oder 20, bei welchem die Zusammensetzung ein nicht in die Flüssigkeitsbeladung aufgenommenes Erweichungsmittel aufweist.

**22.** Verfahren nach Anspruch 14, bei welchem die Flüssigkeitsbeladung ein Sonnenschutzmittel enthält.

**23.** Verfahren nach Anspruch 22, bei welchem das flüssige Sonnenschutzmittel eine Verbindung aufweist, die ausgewählt ist aus Oxybenzon, Amyl-p-Diethylaminobenzoat, Homomethylsalicylat, Octylsalicylat, Mono-p-Aminobenzoat, Octyl-Methoxycinnamat und 2-Ethoxyethyl-p-Methoxycinnamat.

24. Verfahren nach Anspruch 22 oder 23, welches den weiteren Schritt umfaßt, daß ein Erweichungsmittel mit dem flüssigkeits-beladenen Pulver vermischt wird.

25. Verfahren nach Anspruch 13 oder 14, bei welchem ein aktiver Bestandteil in der Flüssigkeitsbeladung enthalten ist.

26. Verfahren nach Anspruch 18, bei welchem ein aktiver Bestandteil in der Dispersionsflüssigkeit enthalten ist.

**Revendications**

1. Un procédé de production d'une poudre chargée de liquide qui est capable de libérer sa charge de liquide quand elle est soumise à des forces de cisaillement, ce procédé comprenant la dissolution d'un polymère cellulosique et d'une charge de liquide dans un solvant polaire pour former une solution et l'évaporation de manière dispersive du solvant de la solution pour former une poudre chargée de liquide caractérisé en ce que le solvant est évaporé de manière dispersive de la solution en atomisant la solution dans une tour et en faisant passer de l'air chauffé ou un gaz inerte par la tour d'une entrée à une sortie à une vitesse et à une température d'entrée suffisantes pour maintenir l'air ou le gaz inerte quittant la tour à une température de sortie supérieure au point de rosée du solvant.

2. Un procédé selon la revendication 1 dans lequel le solvant est volatil et le procédé est en outre caractérisé par le fait que l'on maintient la température d'entrée dans l'intervalle de 30°C à 95°C.

3. Un procédé selon la revendication 1 ou la revendication 2 caractérisé en outre par le fait que l'on maintient une humidité relative d'une valeur inférieure à 40% dans les gaz sortant de la tour.

4. Un procédé selon la revendication 2 dans lequel le solvant polaire est un ester ou un diester à faible poids moléculaire.

5. Un procédé selon la revendication 2 dans lequel le solvant polaire est choisi dans le groupe composé du chlorure de méthylène, du chloroforme, de l'éthanol, de l'acétone, de l'acétate d'éthyle, du carbonate d'éthyle, du formate de méthyle et de leurs mélanges.

6. Un procédé selon la revendication 1 dans lequel le polymère cellulosique est choisi dans le groupe composé des acétates de cellulose, des butyrates de cellulose, des nitrates de cellulose, des propionates de cellulose, des éthyl celluloses et de leurs mélanges discrets ou moléculaires.

7. Un procédé selon la revendication 2 dans lequel le polymère cellulosique est du triacétate de cellulose et le solvant polaire est du chlorure de méthylène.

8. Un procédé selon la revendication 2 dans lequel le polymère cellulosique est de l'éthyl cellulose et le solvant polaire est choisi dans le groupe composé de l'éthanol, du chlorure de méthylène et leurs mélanges.

9. Un procédé selon la revendication 7 dans lequel la tour a une atmosphère contenant de 0 à 40% en volume de chlorure de méthylène.

10. Un procédé selon la revendication 1 dans lequel la charge de liquide constitue jusqu'à 95% en poids de la poudre chargée de liquide.

11. Un procédé selon la revendication 10 dans lequel la charge de liquide constitue de 50 à 95% en poids de la poudre chargée de liquide.

12. Un procédé selon la revendication 8 dans lequel la solution est atomisée par un ou plusieurs injecteurs de haut en bas à partir du sommet de la tour.

13. Un procédé selon la revendication 1, dans lequel la poudre comprend des particules microporeuses ayant une taille de particules dans l'intervalle de 1 à 500 microns et des pores dont la taille est dans l'intervalle de 1 à 500 nanomètres, les pores étant capables de contenir la charge de liquide.

14. Un procédé selon la revendication 13, dans lequel les particules ont une taille moyenne de particules dans l'intervalle de 5 à 100 microns et la proportion de particules ayant une taille supérieure à 150 microns est négligeable.

15. Un procédé selon la revendication 13 ou la revendication 14, comprenant en outre l'étape de mise sous forme d'un gâteau de savon de la poudre chargée de liquide.

16. Un procédé selon la revendication 13 ou la revendication 14, dans lequel la charge de liquide contient un produit pharmaceutique, un agent de filtre solaire, un agent d'hydratation de la peau, un composé parfumant des pâtes dentifrices, un agent antiperspirant, un agent fongicide pour usage sur les pieds, un parfum, une huile pour bébé ou un analgésique topique.

17. Un procédé selon la revendication 15, dans lequel la charge de liquide contient une préparation pour administration orale.

18. Un procédé selon la revendication 13 ou la revendication 14, comprenant en outre l'étape de dispersion de la poudre chargée de liquide dans un liquide dispersant.

19. Un procédé selon la revendication 13 ou la revendication 14, dans lequel la charge de liquide contient un insectifuge.

20. Un procédé selon la revendication 19, dans lequel l'insectifuge est choisi parmi le N,N-diéthyl-m-toluamide et le 2-éthyl-1,3-hexanediol.

21. Un procédé selon la revendication 19 ou la revendication 20, dans lequel la composition comprend en outre un agent émollient non incorporé dans la charge de liquide.

22. Un procédé selon la revendication 14, dans lequel la charge de liquide comprend une préparation de filtre solaire.

23. Un procédé selon la revendication 22, dans lequel la préparation liquide de filtre solaire comprend un composé choisi parmi l'oxybenzone, le p-diéthylaminobenzoate d'amyle, le salicylate d'homométhyle, le salicylate d'octyle, le mono-p-aminobenzoate, le méthoxycinnamate d'octyle, et le 2-éthoxy p-méthoxycinnamate d'éthyle.

24. Un procédé selon la revendication 22 ou la revendication 23, comprenant en outre l'étape de mélange d'un agent émollient avec la poudre chargée de liquide.

25. Un procédé selon la revendication 13 ou la revendication 14, dans lequel un ingrédient actif est contenu dans la charge de liquide.

26. Un procédé selon la revendication 18, dans lequel un ingrédient actif est contenu dans le liquide dispersant.

FIG. 1.